(19) Europäisches Patentamt
European Patent Office
Office européen des brevets

(11) **EP 4 481 671 A1**

(12) **EUROPEAN PATENT APPLICATION**
published in accordance with Art. 153(4) EPC

(43) Date of publication:
**25.12.2024 Bulletin 2024/52**

(21) Application number: **22926203.5**

(22) Date of filing: **07.12.2022**

(51) International Patent Classification (IPC):
**G06T 5/00** (2024.01)      **G06T 7/33** (2017.01)
**G06T 7/73** (2017.01)      **G06T 11/00** (2006.01)
**A61B 6/00** (2024.01)      **A61B 6/14** (2006.01)
**A61B 6/03** (2006.01)      **A61B 5/00** (2006.01)
**A61C 7/00** (2006.01)

(52) Cooperative Patent Classification (CPC):
**G06T 7/33; A61B 5/00; A61B 6/00; A61B 6/03;**
**A61B 6/51; A61C 7/00; G06T 11/00;**
G06T 2207/30036

(86) International application number:
**PCT/KR2022/019837**

(87) International publication number:
**WO 2023/153606 (17.08.2023 Gazette 2023/33)**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority: **14.02.2022 KR 20220018687**

(71) Applicant: **HDX Will Corp.**
**Seoul 03162 (KR)**

(72) Inventors:
• **JUNG, Hong**
  **Seoul 03162 (KR)**
• **LEE, Sung Min**
  **Bucheon-si Gyeonggi-do 14549 (KR)**

(74) Representative: **Murgitroyd & Company**
**165-169 Scotland Street**
**Glasgow G5 8PL (GB)**

(54) **DEVICE AND METHOD FOR RECONSTRUCTING THREE-DIMENSIONAL ORAL SCAN DATA BY USING COMPUTED TOMOGRAPHY IMAGE**

(57)     The present invention relates to a device and a method for reconstructing three-dimensional oral scan data by using a computed tomography (CT) image. According to an embodiment of the present invention, reconstruction is performed by generating 3D coordinate information and 3D feature points from a CT image without geometric distortion and locally matching the CT image and scan key frames obtained from a scanner, so that errors due to accumulated matching of the scan key frames can be reduced, thereby reducing geometric distortion of an oral scan model.

FIG. 1

**Description**

[Technical Field]

The present invention relates to a three-dimensional oral scan data reconstruction technology, and more particularly, to a device and a method for reconstructing three-dimensional oral scan data, which can reduce geometric distortion of a scan model by using a computed tomography (CT) image.

[Background Art]

In a dental field, including orthodontics and implants, establishing an accurate diagnostic plan and treatment are very important. For this purpose, a 3D computed tomography image (hereinafter, referred to as 'CT images') and 3D oral scan data are used. The CT image as an image reconstructed by taking an entire tooth in an oral cavity at once has an advantage in that tooth root information can be known and geometric distortion does not occur, and since the 3D oral scan data has an advantage of having higher tooth crown precision than the CT image, both the CT image and the 3D oral scan data are generally used for diagnosis and treatment.

However, the 3D oral scan data has a disadvantage of causing the geometric distortion because the 3D oral scan data is an image that is reconstructed by stitching together multiple pieces of local data within the oral cavity using a mathematical algorithm. In other words, the 3D oral scan data is an image reconstructed by generating multiple scan frames by locally scanning each part of the oral cavity using an oral scanner, and then matching point clouds included in each generated scan frame into one in a 3D coordinate system. Accordingly, even if a matching error between one frame pair is small, errors are accumulated when matching all frames, and the accumulated errors cause the geometric distortion in a 3D oral model, reducing the reliability of the 3D oral scan data.

Therefore, research and development on data reconstruction technology that can reduce the geometric distortion of the scan model is required to establish accurate diagnostic plans and treatments in the dental field, including orthodontics and implants.

[Prior Art Document]

[Patent Document]

(Patent Document 1) KR 10-2273437 B1, June 30, 2021

[Disclosure]

[Technical Problem]

An object of the present invention is to provide a device and a method for reconstructing 3D oral scan data, which may reduce geometric distortion of a scan model by using a CT image with no geometric distortion.

Further, another object of the present invention is to provide an oral scanner and a computed tomography apparatus which may reduce the geometric distortion of the scan model by using the CT image with no geometric distortion.

Further, yet another object of the present invention is to provide a computer program stored in a computer-readable recording medium implementing a method for reconstructing 3D oral scan data, which may reduce the geometric distortion of the scan model by using the CT image, and a recording medium storing the program.

In addition, the present invention is not limited to the above-described objects, and besides, various objects may be additionally provided through techniques described through embodiments and claims described later.

[Technical Solution]

In order to achieve the object, an aspect of the present invention provides a method for reconstructing 3D oral scan data by using a computed tomography (CT) image and scan data of which coordinates are matched based on 3D feature points of a detected CT image and scan data, which includes: (a) a process of matching scan key frames of the scan data initially positioned by the 3D feature points, and obtaining a rigid transform of the scan key frames to minimize a 3D distance between the scan key frames and the CT image; and (b) a process of reconstructing a 3D oral scan model by correcting and rematching 3D coordinate information of an original scan key frame by using the obtained rigid transform of the scan key frames.

Further, in the process (a), if the number of scan frames for a scan frame set $S = \{s_1, s_2, \cdots\}$ is $|S|$, a rigid transform

$$\left\{ T_i \right\}_{i=1}^{|S|}$$ of each scan frame that minimizes a loss L may be obtained as in [Equation] below.

$$L\left(\left\{ T_i \right\}_{i=1}^{|S|}\right) = \sum_{i=1}^{|S|} w_{1,i} \sum_{j=1,(i,j)}^{|S|} \sum_{\in C(i,j)} \| T p_{i,k_1} - T p_{j,i} \|^2 + \sum_{i=1}^{|S|} w_{2,i} \sum_{k_2=0}^{N_i} \| T p_{i,k_2} - c_{i,k_2} \|^2$$

Where $p_{i,k}$ represents a point corresponding an $i$-th scan key frame, $C(i,j)$ represents a set of all points that are shared between an $i$-th key frame and a $j$-th key frame, $c_{i,k}$ represents a point of 3D coordinate information of the CT image corresponding to $p_{i,k}$, $N_i$ represents the number of points where $p_{i,k}$ and $c_{i,k}$ correspond to each other, and $w_{1,i}$ and $w_{2,i}$ represent weights corresponding to respective losses.

Further, in the process (a), when $w_{1,i}$ becomes larger upon matching the scan key frames of the scan data, matching the scan key frames may be emphasized, and when $w_{2,i}$ becomes larger, matching the scan key frame and the CT image may be emphasized.

In addition, in the process (a), by comparison with a rigid transform $T_i$ in which $L(T_i)$ is minimal with respect to fixed $w_{1,i}$ and $w_{2,i}$, a rigid transform obtained by increasing only $w_{1,i}$ may decrease a matching error between scan key frames instead of increasing the matching error between the scan key frame and the CT image, and in contrast, a rigid transform obtained by increasing only $w_{2,i}$ may decrease the matching error between the scan key frame and the CT image instead of increasing the matching error between the scan key frames.

In addition, in the process (a), when artifact is severe in the CT image, a size of $w_{2,i}$ is partially decreased or is set to 0, and a size of $w_{1,i}$ is increased to reduce an influence of the CT image when matching the scan key frames, and in contrast, when the matching error between the scan key frames is large, the size of $w_{1,i}$ is decreased or is 0, or the size of $w_{2,i}$ is increased to increase an influence of the CT image, thereby reducing the matching error between the scan key frames.

Further, in order to achieve the object, another aspect of the present invention provides a method for reconstructing 3D oral scan data by using a computed tomography image, which includes: (a) a process of generating 3D coordinate information from a computed tomography (CT) image; (b) a process of detecting 3D coordinate information of the CT image and a 3D feature point of scan data; (c) a process of matching the 3D coordinate information of the CT image and coordinates of the scan data by using the 3D feature points; (d) a process of matching scan key frames of the scan data initially positioned by the 3D feature points, and obtaining a rigid transform of the scan key frames to minimize a 3D distance between the scan key frames and the CT image; and (e) a process of reconstructing a 3D oral scan model by correcting and rematching 3D coordinate information of an original scan key frame by using the obtained rigid transform of the scan key frames.

Further, step (b) above may include (b-1) a process of generating a 2D rendering image for the 3D coordinate information of the scan data and the CT image; (b-2) a process of detecting adjacent 2D points between teeth in the 2D rendering image; (b-3) a process of detecting adjacent 3D points between teeth based on the detected 2D points; and (b-4) a process of obtaining a direction perpendicular to a virtual straight line passing through two adjacent detected 3D points, and obtaining a center point which becomes a center between two adjacent 3D points in the obtained direction perpendicular to the virtual straight line, and then sampling points on the 3D coordinate information in the perpendicular direction obtained from two adjacent 3D points and the center point to detect the 3D coordinate information of the CT image and the 3D feature point of the scan data.

Further, in the process (c), a rotation matrix R and a translation vector t are obtained by using [Equation 1] below by using the detected 3D feature pointsas an initial value, and coordinates of the scan data and the CT image are approximately matched, and the coordinates of the scan data and the CT image are matched by using [Equation] below with respect to all 3D coordinate information generated by the CT image and all points of the scan data again to minimize errors.

[Equation]

$$E(R,t) = \| C - (RD + t) \|$$

Where 'C' represents the 3D coordinate information generated from the CT image, and 'D' represents the scan data.

Further, in the process (c), if the numbers of 3D feature points of the scan data and the CT image are n with respect to all 3D coordinate information C and scan data D generated by the CT image, the scan data is transformed into $R_1 D + t_1$ by

obtaining an initial rigid transform $R_1$, $t_1$ of minimizing the error $E(R_1,t_1)$ by using [Equation 1] below with respect to the 3D feature points $\{c_i\}_{i=0}^{n} \subseteq C$ of the CT image and the 3D feature points $\{d_i\}_{i=0}^{n} \subseteq D$ of the scan data to approximately match the coordinates of the scan data and the CT image, and coordinate information of the scan data D is transformed into $R(R_1 D + t_1) + t$ by obtaining the rigid transform R,t of minimizing the error $E(R,t)$ by using [Equation 2] below with respect to 3D coordinate information C generated by the CT image and all points $R_1 D + t_1$ of coordinate-shifted scan data to match the coordinates of the scan data and the CT image.

[Equation 1]

$$E(R_1, t_1) = \sum_{i=0}^{n} \| c_i - (R_1 d_i + t_1) \|$$

[Equation 2]

$$E(R, t) = \| C - (R(R_1 D + t_1) + t) \|$$

Further, in the process (d), if the number of scan frames for a scan frame set $S = \{s_1, s_2, \cdots\}$ is $|S|$, a rigid transform $\{T_i\}_{i=1}^{|S|}$ of each scan frame that minimizes a loss L may be obtained as in [Equation] below.

[Equation]

$$L(\{T_i\}_{i=1}^{|S|}) = \sum_{i=1}^{|S|} w_{1,i} \sum_{j=1}^{|S|} \sum_{(k_1, l) \in C(i,j)} \| T_i p_{i,k_1} - T_j p_{j,l} \|^2 + \sum_{i=1}^{|S|} w_{2,i} \sum_{k_2=0}^{N_i} \| T_i p_{i,k_2} - c_{i,k_2} \|^2$$

Where $p_{i,k}$ represents a point corresponding an $i$-th scan key frame, $C(i,j)$ represents a set of all points that are shared between an $i$-th key frame and a $j$-th key frame, $c_{i,k}$ represents a point of 3D coordinate information of the CT image corresponding to $p_{i,k}$, $N_i$ represents the number of points where $p_{i,k}$ and $c_{i,k}$ correspond to each other, and $w_{1,i}$ and $w_{2,i}$ represent weights corresponding to respective losses.

Further, in the process (d), when $w_{1,i}$ becomes larger upon matching the scan key frames of the scan data, matching the scan key frames may be emphasized, and when $w_{2,i}$ becomes larger, matching the scan key frame and the CT image may be emphasized.

In addition, in the process (d), by comparison with a rigid transform $T_i$ in which $L(T_i)$ is minimal with respect to fixed $w_{1,i}$ and $w_{2,i}$, in the same condition, a rigid transform obtained by increasing only $w_{1,i}$ may decrease a matching error between scan key frames instead of increasing the matching error between the scan key frame and the CT image, and in contrast, a rigid transform obtained by increasing only $w_{2,i}$ may decrease the matching error between the scan key frame and the CT image instead of increasing the matching error between the scan key frames.

In addition, in the process (d), when artifact is severe in the CT image, a size of $w_{2,i}$ is partially decreased or is set to 0, and a size of $w_{1,i}$ is increased to reduce an influence of the CT image when matching the scan key frames, and in contrast, when the matching error between the scan key frames is large, the size of $w_{1,i}$ is decreased or is 0, or the size of $w_{2,i}$ is increased to increase an influence of the CT image, thereby reducing the matching error between the scan key frames.

Further, in order to achieve the object, yet another aspect of the present invention provides a device for reconstructing 3D oral scan data by using a computed tomography image, which includes: a 3D coordinate information generation unit generating 3D coordinate information from a computed tomography (CT) image; a 3D feature point detection unit detecting 3D coordinate information of the CT image and a 3D feature point of scan data; a coordinate matching unit matching the 3D coordinate information of the CT image and coordinates of the scan data by using the 3D feature points; a rematching and rigid transform calculation unit matching scan key frames of the scan data initially positioned by the 3D feature points, and obtaining a rigid transform of the scan key frames to minimize a 3D distance between the scan key frames and the CT image; and an oral scan model reconstruction unit reconstructing a 3D oral scan model by correcting and rematching 3D coordinate information of an original scan key frame by using the obtained rigid transform of the scan key frames.

Further, the 3D feature point detection unit may generate a 2D rendering image for the 3D coordinate information of the scan data and the CT image, detect adjacent 2D points between teeth in the 2D rendering image, detect adjacent 3D points between teeth based on the detected 2D points, and obtain a direction perpendicular to a virtual straight line passing through two adjacent detected 3D points, and obtain a center point which becomes a center between two adjacent 3D points in the obtained direction perpendicular to the virtual straight line, and then sample points on the 3D coordinate information in the perpendicular direction obtained from two adjacent 3D points and the center point to detect the 3D coordinate information of the CT image and the 3D feature point of the scan data.

Further, the coordinate matching unit obtains a rotation matrix R and a translation vector t by using [Equation] below by using the detected 3D feature points as an initial value, and approximately matches coordinates of the scan data and the CT image, and matches the coordinates of the scan data and the CT image by using [Equation] below with respect to all 3D coordinate information generated by the CT image and all points of the scan data again to minimize errors.

[Equation]

$$E(R,t) = \|C - (RD + t)\|$$

Where 'C' represents the 3D coordinate information generated from the CT image, and 'D' represents the scan data.

Further, if the numbers of 3D feature points of the scan data and the CT image are n with respect to all 3D coordinate information C and scan data D generated by the CT image, the coordinate matching unit transforms the scan data into $R_1 D + t_1$ by obtaining an initial rigid transform $R_1, t_1$ of minimizing the error $E(R_1, t_1)$ by using [Equation 1] below with respect to the 3D feature points $\{c_i\}_{i=0}^n \subseteq C$ of the CT image and the 3D feature points $\{d_i\}_{i=0}^n \subseteq D$ of the scan data to approximately match the coordinates of the scan data and the CT image, and transforms coordinate information of the scan data D into $R(R_1 D + t_1) + t$ by obtaining the rigid transform R,t of minimizing the error $E(R,t)$ by using [Equation 2] below with respect to 3D coordinate information C generated by the CT image and all points $R_1 D + t_1$ of coordinate-shifted scan data to match the coordinates of the scan data and the CT image.

[Equation 1]

$$E(R_1, t_1) = \sum_{i=0}^{n} \|c_i - (R_1 d_i + t_1)\|$$

[Equation 2]

$$E(R,t) = \|C - (R(R_1 D + t_1) + t)\|$$

Further, if the number of scan frames for a scan frame set $S = \{s_1, s_2, \cdots\}$ is $|S|$, the rematching and rigid transform calculation unit may obtain a rigid transform $\{T_i\}_{i=1}^{|S|}$ of each scan frame that minimizes a loss L as in [Equation] below.

[Equation]

$$L(\{T_i\}_{i=1}^{|S|}) = \sum_{i=1}^{|S|} w_1 \sum_{j=1}^{|S|} \sum_{k,l \in C(i,j)} \|T p_{i,k} - T p_{j,l}\|^2 + \sum_{i=1}^{|S|} w_2 \sum_{k=0}^{N_i} \|T p_{i,k} - c_{i,k}\|^2$$

Where $p_{i,k}$ represents a point corresponding an $i$-th scan key frame, $C(i,j)$ represents a set of all points that are shared between an $i$-th key frame and a $j$-th key frame, $c_{i,k}$ represents a point of 3D coordinate information of the CT image corresponding to $p_{i,k}$, $N_i$ represents the number of points where $p_{i,k}$ and $c_{i,k}$ correspond to each other, and $w_{1,i}$ and $w_{2,i}$ represent weights corresponding to respective losses.

Further, the rematching and rigid transform calculation unit may emphasize matching the scan key frames when $w_{1,i}$ becomes larger upon matching the scan key frames of the scan data, and emphasize matching the scan key frame and the CT image when $w_{2,i}$ becomes larger.

Further, by comparison with a rigid transform $T_i$ in which $L(T_i)$ is minimal with respect to fixed $w_{1,i}$ and $w_{2,i}$, in the same condition, the rematching and rigid transform calculation unit may decrease a matching error between scan key frames instead of increasing the matching error between the scan key frame and the CT image in a rigid transform obtained by increasing only $w_{1,i}$, and in contrast, decrease the matching error between the scan key frame and the CT image instead of increasing the matching error between the scan key frames in a rigid transform obtained by increasing only $w_{2,i}$.

Further, the rematching and rigid transform calculation unit partially decreases a size of $w_{2,i}$ or sets the size of $w_{2,i}$ to 0, and increases a size of $w_{1,i}$ to reduce an influence of the CT image when matching the scan key frames when artifact is severe in the CT image, and in contrast, decreases the size of $w_{1,i}$ or set the size of $w_{1,i}$ to 0, and increases the size of $w_{2,i}$ to increase an influence of the CT image when the matching error between the scan key frames is large, thereby reducing the matching error between the scan key frames.

Further, in order to achieve the object, still yet another aspect of the present invention provides an oral scanner including the device for reconstructing 3D oral scan data by using the computed tomography image described above.

In addition, in order to achieve the object, still yet another aspect of the present invention provides a computed tomography (CT) apparatus including the device for reconstructing 3D oral scan data by using a computed tomography image.

Further, in order to achieve the object, still yet another aspect of the present invention provides a computer-readable recording medium storing a program for implementing the method for reconstructing 3D oral scan data by using the computed tomography image described above.

In addition, in order to achieve the object, still yet another aspect of the present invention provides a computer program stored in a computer-readable recording medium for implementing the method for reconstructing 3D oral scan data by using a computed tomography image.

[Advantageous Effects]

As described above, by the device and the method for reconstructing 3D oral scan data by using a computed tomography image according to the embodiments of the present invention, accumulated matching errors of scan frames are reduced by rematching the scan frames according to a geometric structure of a CT image by using a CT image with no geometric distortion to reduce the geometric distortion of an oral scan model.

[Description of Drawings]

FIG. 1 is a block diagram illustrating a device for reconstructing 3D oral scan data according to an embodiment of the present invention.

FIG. 2 is a flowchart illustrating a method for reconstructing 3D oral scan data according to an embodiment of the present invention.

FIG. 3 is a diagram illustrating a 3D feature point detection process illustrated in FIG. 2 as an image.

FIG. 4 is a flowchart illustrating a detailed process of the 3D feature point detection process illustrated in FIG. 2.

FIG. 5 is a diagram illustrating the detailed process of the 3D feature point detection process illustrated in FIG. 4 as the image.

FIG. 6 is a flowchart illustrating a process of detecting a 3D feature point for 3D coordinate information of a CT image according to the present invention.

FIG. 7 is a diagram illustrating the detection process illustrated in FIG. 6 as the image.

FIG. 8 is a diagram illustrating a process in which scan key frames are matched according to the CT image of the present invention as the image.

FIG. 9 is a diagram illustrating an example of a CT image with metal artifact.

FIG. 10 is a diagram illustrating a process of matching the 3D feature point of the CT image with each scan data according to the present invention as the image.

[Best Mode]

Hereinafter, advantages and features of the present invention, and methods for accomplishing the same will be more clearly understood from embodiments described in detail below with reference to the accompanying drawings. However, the present invention is not limited to the embodiments set forth below, and will be embodied in various different forms. The embodiments are just for rendering the disclosure of the present invention complete and are set forth to provide a complete understanding of the scope of the invention to a person with ordinary skill in the technical field to which the present invention pertains, and the present invention will be defined by the scope of the claims.

Further, it is also to be understood that the terminology used herein is for the purpose of describing embodiments only and is not intended to limit the present invention. In addition, in this specification, singular forms include even plural forms unless the context clearly indicates otherwise. For example, 'include' (or 'comprise') and/or 'including' (or 'comprising') used in the specification can add the mentioned components and processes. Throughout the whole specification, the same reference numerals denote the same elements. 'And/or' includes each and every combination of one or more of the mentioned items.

Further, unless otherwise defined, all terms (including technical and scientific terms) used in the present specification may be used as the meaning which may be commonly understood by the person with ordinary skill in the art, to which the present invention pertains. Terms defined in commonly used dictionaries should not be interpreted in an idealized or excessive sense unless expressly and specifically defined.

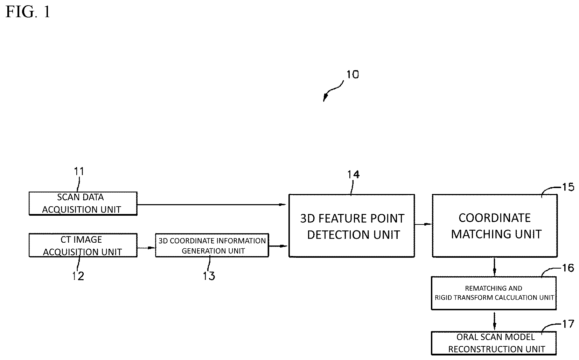

FIG. 1 is a block diagram illustrating a device for reconstructing 3D oral scan data according to an embodiment of the present invention.

Referring to FIG. 1, the 3D oral scan data reconstruction device 10 according to an embodiment of the present invention includes a scan data acquisition unit 11, a CT image acquisition unit 12, a 3D coordinate information generation unit 13, a 3D feature point detection unit 14, a coordinate matching unit 15, a rematching and rigid transform calculation unit 16, and an oral scan model reconstruction unit 17 in order to reconstruct oral scan data by locally matching scan key frames of the oral scan data with a CT image with no geometric distortion.

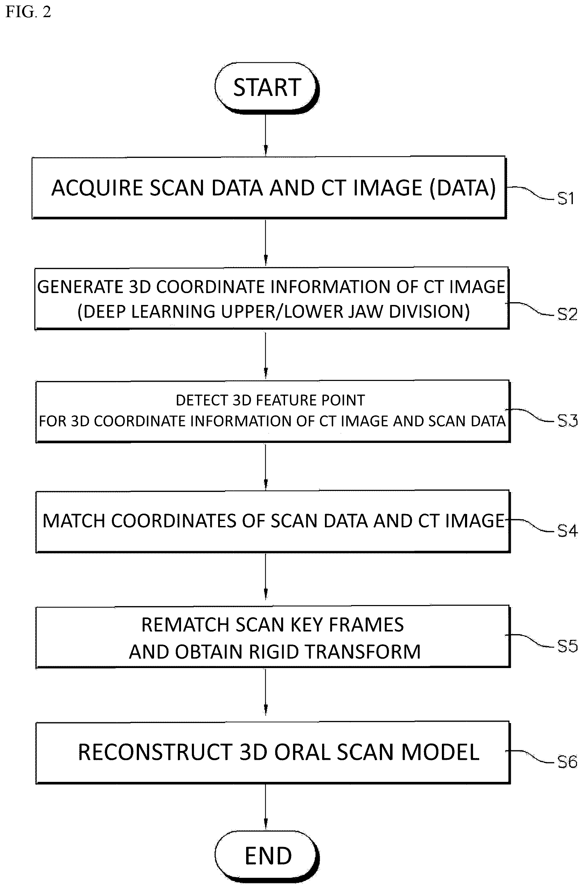

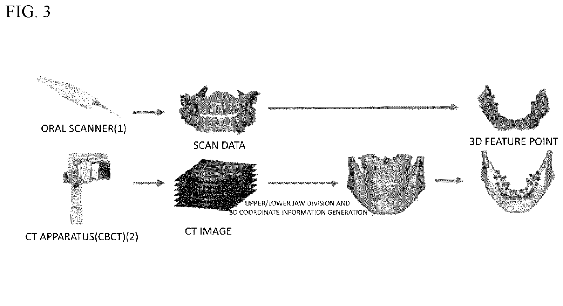

FIG. 2 is a flowchart illustrating a method for reconstructing 3D oral scan data according to an embodiment of the present invention, and FIG. 3 is a diagram illustrating a 3D feature point detection process illustrated in FIG. 2 as an image.

As illustrated in FIGS. 1 to 3, the scan data acquisition unit 11 acquires scan data (S1). At this time, the scan data acquisition unit 11 may be an oral scanner 1 that acquires scan data by locally scanning the inside of the patient's oral cavity. Alternatively, the scan data acquisition unit 11 also wiredly or wirelessly communicates with the oral scanner 1 to receive and acquire the scan data obtained through the oral scanner 1 through wired or wireless communication. Here, the scan data may include a plurality of scan frames.

The CT image acquisition unit 12 which acquires a CT image obtained by capturing the inside of the patient's oral cavity may be, for example, CT apparatus 2. Alternatively, the CT image of the inside of the patient's oral cavity captured through the CT apparatus 2 may be provided and acquired from the CT apparatus 2 through the wired and wireless communication. At this time, the CT apparatus 2 may be, for example, cone beam computed tomography (CBCT) apparatus.

The 3D coordinate information generation unit 13 generates 3D coordinate information of the CT image acquired by the CT image acquisition unit 12 (S2). For example, the 3D coordinate information generation unit 13 divides the CT image into upper and lower jaws using deep learning, and then generates 3D coordinate information for the upper and lower jaws of the divided CT image, respectively. At this time, the 3D coordinate information may mean a depth map, a 3D point cloud, a 3D mesh, or the like.

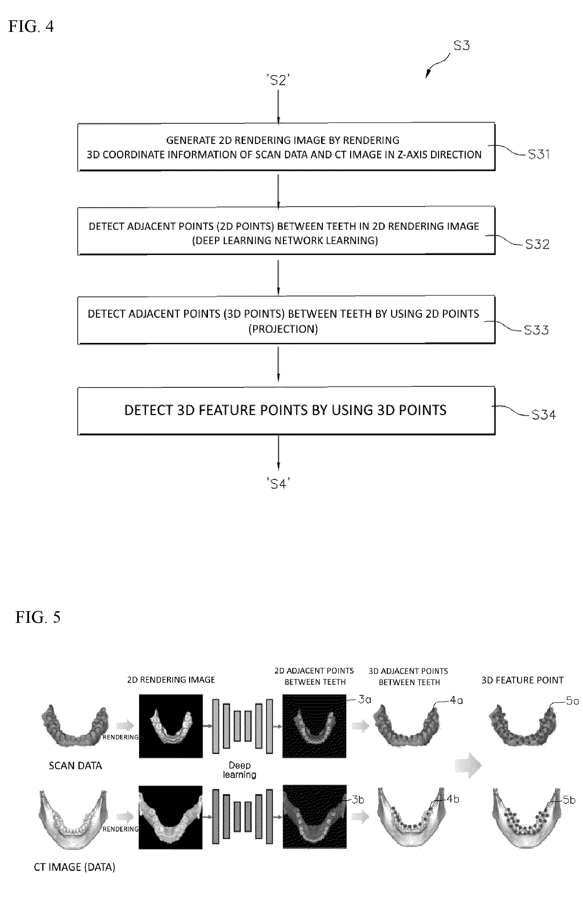

The 3D feature point detection unit 14 detects a 3D feature point for the 3D coordinate information of the CT image generated by the 3D coordinate information generation unit 13 and a 3D feature point for the scan data acquired by the scan data acquisition unit 11 (S3). The 3D feature point detection process S3 is illustrated in FIGS. 4 and 5.

FIG. 4 is a flowchart illustrating a detailed process of the 3D feature point detection process illustrated in FIG. 2, and FIG. 5 is a diagram illustrating the detailed process of the 3D feature point detection process illustrated in FIG. 4 as the image.

The 3D feature point detection process S3 is illustrated as below with reference to FIGS. 4 and 5.

Rendering is performed in a z-axis direction on the 3D coordinate information of the acquired scan data and the generated CT image to generate a 2D rendered image for the scan data and the CT image (S31).

Subsequently, adjacent points 3a and 3b (hereinafter, referred to as '2D point') between teeth are detected in the 2D rendering images generated from the 3D coordinate information of the scan data and the CT image, respectively in the process S31 (S32). For example, the 2D point detection process S32 may be detected by using a deep learning network learning of finding adjacent points between teeth, i.e., 2D points 3a and 3b in the 2D rendering image.

Subsequently, 3D inter-teeth adjacent points 4a and 4b (hereinafter, referred to as '3D point') are detected by using the 2D points 3a and 3b detected in the process S32 (S33). For example, the 2D points 3a and 3b detected through deep learning are projected to original 3D data (scan data and CT image) to detect the 3D points 4a and 4b for the scan data and the CT image (S33).

Subsequently, a 3D point 5a for the scan data and a 3D feature point 5b for the 3D coordinate information of the CT image are detected by using the respective 3D points 4a and 4b detected in the process S33 (S34).

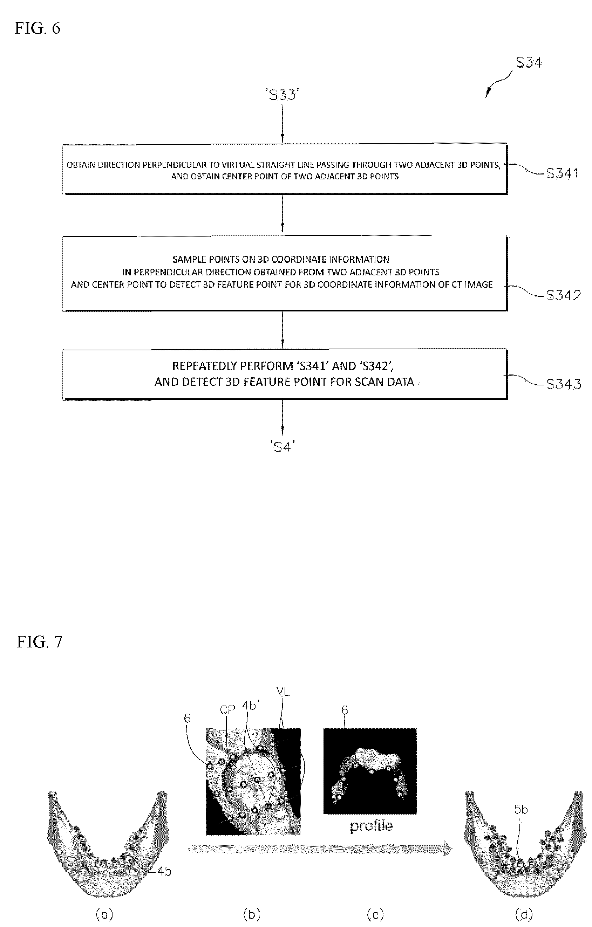

The process S34 of detecting the 3D feature points 5a and 5b using the 3D points 4a and 4b is illustrated in FIGS. 6 and 7.

FIG. 6 is a flowchart illustrating a process of detecting a 3D feature point for 3D coordinate information of a CT image according to the present invention, and FIG. 7 is a diagram illustrating the detection process illustrated in FIG. 6 as the image. Here, (a) of FIG. 7 is a diagram illustrating 3D points for the CT image, (b) of FIG. 7 is a partial enlarged diagram of (a) of FIG. 7, (c) of FIG. 7 is a cross-sectional view of (b) of FIG. 7, and (d) of FIG. 7 is a diagram illustrating 3D feature points for the CT image.

As illustrated in FIG. 6 and (b) of FIG. 7, after obtaining a direction perpendicular to a virtual straight line VL passing through two adjacent 3D points 4b' on the CT image, a center point Cp which becomes a center between two adjacent 3D points 4b' in the obtained direction perpendicular to the virtual straight line VL is obtained (S341).

Subsequently, as illustrated in (b) of FIG. 7 and (d) of FIG. 7, a 3D feature point 5b for the 3D coordinate information of the CT image is detected by sampling two adjacent 3D points 4b' and points 6 on the 3D coordinate information of the CT image in a direction perpendicular to the obtained center point cp (S342).

Subsequently, by the same method as the above-described processes S341 and s342, the processes are repeatedly performed to detect the 3D feature point 5a for the scan data (S343). For example, in the scan data, the direction perpendicular to the virtual straight line passing through two adjacent 3D points is obtained, and then the center point which becomes the center between two adjacent 3D points in the obtained direction perpendicular to the virtual straight line is obtained, and two adjacent 3D points, and the points on the 3D coordinate information of the scan data in the direction perpendicular to the center point are sampled to detect the 3D feature point 5a for the scan data.

In the present invention, the 3D feature point for the 3D coordinate information of the CT image is detected, and then the 3D feature point for the scan data is detected, but this is an example, and the process of detecting the 3D feature point for the scan data may be performed simultaneously with the process of detecting the 3D feature point for the 3D coordinate information of the CT image, or also performed before the process. In other words, the order is not limited.

Then, when the 3D feature points 5a and 5b for the 3D coordinate information of the scan data and the CT image are detected, as illustrated in FIGS. 1 and 2, the coordinate matching unit 15 matches coordinates of the scan data and the CT image by using the 3D feature points 5a and 5b detected through the 3D feature point detection unit 14 (S4). The coordinates of the scan data and the CT image may be matched with each other by the following method.

In the process of matching the coordinates of the scan data and the CT image, first, a rotation matrix R and a translation vector t in which an error $E(R,t)$ becomes minimal are obtained as in [Equation 1] below by using the 3D feature point as an initial value.

[Equation 1]

$$E(R,t) = \|C - (RD + t)\|$$

Where 'C' represents the 3D coordinate information generated from the CT image, and 'D' represents the scan data.

In other words, the process of matching the coordinates of the scan data and the CT image is to approximately match the coordinates of the scan data and the CT image by obtaining the approximate rotation matrix R and translation vector t using [Equation 1] above based on the 3D feature point, and matches the coordinates of the scan data and the CT image by using [Equation 1] above with respect to all 3D coordinate information (including the 3D feature point) generated by the CT image and all points (including the 3D feature point) of the scan data again to minimize errors.

For example, if the numbers of 3D feature points of the scan data and the CT image are n with respect to all 3D coordinate information C and scan data D generated by the CT image, the scan data is transformed into $R_1 D + t_1$ by obtaining an initial rigid transform $R_1, t_1$ of minimizing the error $E(R_1, t_1)$ by using [Equation 2] below with respect to the 3D feature points $\{c_i\}_{i=0}^{n} \subseteq C$ of the CT image and the 3D feature points $\{d_i\}_{i=0}^{n} \subseteq D$ of the scan data to approximately match the coordinates of the scan data and the CT image. In addition, coordinate information of the scan data D is transformed into $R(R_1 D + t_1) + t$ by obtaining the rigid transform R,t of minimizing the error $E(R,t)$ by using [Equation 3] below with respect to 3D coordinate information C generated by the CT image and all points $R_1 D + t_1$ of coordinate-shifted scan data to match the coordinates of the scan data and the CT image.

[Equation 2]

$$E(R_1, t_1) = \sum_{i=0}^{n} \| c_i - (R_1 d_i + t_1) \|$$

[Equation 3]

$$E(R, t) = \| C - (R(R_1 D + t_1) + t) \|$$

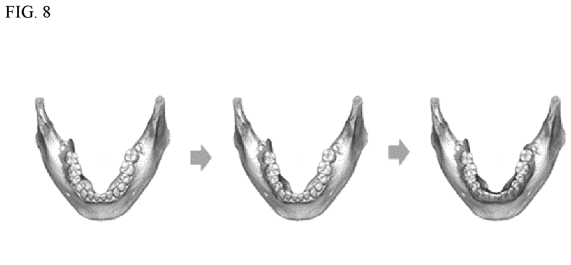

FIG. 8 is a diagram illustrating a process in which scan key frames are matched according to the CT image of the present invention as the image.

As illustrated in FIGS. 1, 2, and 8, the rematching and rigid transform calculation unit 16 matches scan key frames of the scan data initially positioned by the detected 3D feature points, and simultaneously obtains a rigid transform of the scan key frames as in [Equation 4] below so as to minimize a 3D distance between the scan key frames and the CT images (S5).

In this specification, the scan key frame is a term used to expand a semantic range of the scan frame. For example, the scan key frame may be one scan data or data representing adjacent scan frames. Alternatively, the scan data may also be reprojected data. Here, if the scan key frame is one scan frame and the number of key frames is equal to the total number of frames, this means that all frames are matched, and if it is a frame made by combining several scan frames into one rather than a single frame unit (a result obtained by scanning a predetermined range), this means that frame sets that have already been matched for each section are matched between sets. The reprojected data means that scan data that has already been matched once is reprojected toward a camera again to create and use a new frame.

This method may reduce the accumulated matching errors of the scan frames because the scan frames are matched according to the geometric structure of the CT image. A method for obtaining a rigid transform that satisfies this is as follows.

For example, if the number of scan frames for a scan frame set $S = \{s_1, s_2, \cdots \}$ is $|S|$, a rigid transform $\{T_i\}_{i=1}^{|S|}$ of each scan frame that minimizes a loss L below may be obtained as in [Equation 4] below.

[Equation 4]

$$L(\{T_i\}_{i=1}^{|S|}) = \sum_{i=1}^{|S|} w_{1,i} \sum_{j=1(i,j)}^{|S|} \sum_{j \in C(i,j)} \| T p_{i,k_i} - T p_{j,l} \|^2 + \sum_{i=1}^{|S|} w_{2,i} \sum_{k_i=0}^{N_i} \| T p_{i,k_i} - c_{i,k_i} \|^2$$

Where $p_{i,k}$ represents a point corresponding an $i$-th scan key frame, $C(i,j)$ represents a set of all points that are shared between an $i$-th key frame and a $j$-th key frame, $c_{i,k}$ represents a point of 3D coordinate information of the CT image corresponding to $p_{i,k}$, $N_i$ represents the number of points where $p_{i,k}$ and $c_{i,k}$ correspond to each other, and $w_{1,i}$ and $w_{2,i}$ represent weights corresponding to respective losses.

Further, when $w_{1,i}$ increases, matching the scan key frames may be emphasized, and when $w_{2,i}$ increases, matching the scan key frame and the CT image may be emphasized. In some cases, each weight may be '0'. By comparison with a rigid transform $T_i$ in which $L(T_i)$ is minimal with respect to fixed $w_{1,i}$ and $w_{2,i}$ in the same condition, a rigid transform obtained by increasing only $w_{1,i}$ decreases a matching error between scan key frames instead of increasing the matching error between the scan key frame and the CT image. In contrast, a rigid transform obtained by increasing only $w_{2,i}$ decreases the matching error between the scan key frame and the CT image instead of increasing the matching error between the scan key frames.

Meanwhile, a direction in which a 3D distance between scan key frame data and CT image data corresponding thereto is minimal means that the scan key frame data and the CT image data are close to each other.

A method for minimizing the 3D distance between the scan key frame data and the CT image data may minimize a 3D difference between coordinates of points $p_k$ on the scan key frame and points $c_k$ on the CT image corresponding thereto as in [Equation 5] below.

[Equation 5]

$$\min \sum_{k=0} \|p_k - c_k\|$$

Further, as in [Equation 6] below, a value by inner product of a normal vector $n_k$ of respective points with respect to a difference for 3D coordinates of respective points of the scan key frame data and the CT image data may also be minimized. Here, the normal vector means a normal vector for points on target data to which data is to be moved.

[Equation 6]

$$\min \sum_{k=0} \|n_k (p_k - c_k)\|$$

Further, as in [Equation 7] below, a difference between depth map points $d^{p_k}$ of the scan key frame and depth map points $d^{c_k}$ on the CT image may also be minimized.

[Equation 7]

$$\min \sum_{k=0} \|(d^{p_k} - d^{c_k})\|$$

As such, the method for minimizing the 3D distance between the scan key frame data and the CT image data may be calculated by various methods.

FIG. 9 is a diagram illustrating an example of a CT image with metal artifact.

As illustrated in FIG. 9, a tooth including metal in the tooth CT image has severe metal artifact, but a tooth which does not include the metal has almost no artifact. When CT artifact is severe, a size of $w_{2,i}$ is partially decreased or is set to 0, and a size of $w_{1,i}$ is increased to reduce an influence of the CT image when matching the scan key frames. In contrast, when the matching error between the scan key frames is large, the size of $w_{1,i}$ is decreased or is 0, and the size of $w_{2,i}$ is increased to increase an influence of the CT image, thereby reducing the matching error between the scan key frames.

Therefore, in the present invention, like the rigid transform obtained in [Equation 4] above, by using a rigid transform considering both a relationship ($w_{2,i}$ part) with the CT image and a relationship ($w_{1,i}$ part) between the scan frames, the 3D coordinate information of the scan frame is corrected and rematched to supplement an artifact problem by emphasizing a matching weight between the scan frames when the artifact of the CT image (an image in which an unnecessary part is captured when capturing the CT image) is severe.

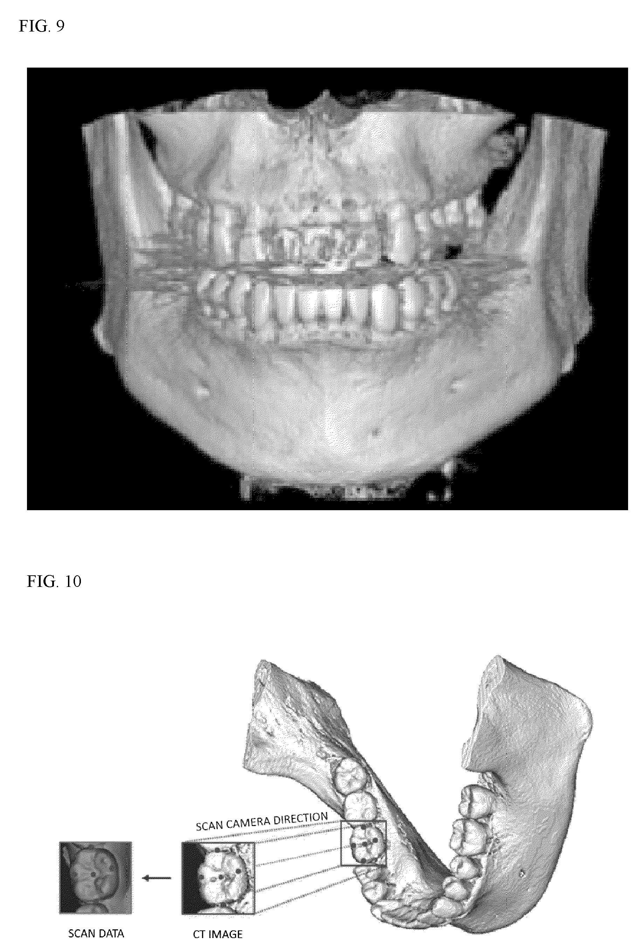

FIG. 10 is a diagram illustrating a process of matching the 3D feature point of the CT image with each scan data according to the present invention as the image.

As illustrated in FIG. 10, after the coordinates of the CT image and the scan data are matched with each other, a similarity (an inner product of the scan data and the CT image, shift, etc.) between the CT image and the scan data is measured, and a 3D feature point for the 3D coordinate information of the CT image detected by the 3D feature point detection unit 14 is matched with each scan data to obtain the 3D feature point of the scan data again, and matching may also be performed by the rematching and rigid transform calculation unit 16 by using only the 3D feature point of the scan data obtained again.

As illustrated in FIGS. 1 and 2, the oral scan model reconstruction unit 17 reconstructs a 3D oral scan model by correcting and matching the existing scan key frames by using the rigid transform obtained through the rematching and rigid transform calculation unit 16 (S6). That is, the oral scan model reconstruction unit 17 reconstructs a 3D oral scan model by rematching 3D coordinate information of an original scan key frame by using the rigid transform obtained through [Equation 4] above through the rematching and rigid transform calculation unit 16.

Meanwhile, the device and the method for reconstructing the 3D oral scan data according to the embodiments of the present invention may be provided as one module or software form in the oral scanner or CT apparatus. For example, the oral scanner includes the components of FIG. 1 to reconstruct the 3D oral scan model by receiving the CT image from the CT apparatus, and the CT apparatus includes the components of FIG. 1 to reconstruct the 3D oral scan model by receiving the scan data from the oral scanner.

Further, the device and the method for reconstructing the 3D oral scan data according to the embodiments of the present invention may be implemented, for example, as a form (or a computer program product) of a recording medium executable by a computer, such as a program module stored in a computer-readable recording medium and executed by the computer. Here, the computer-readable recording medium may include computer storage media (e.g., a memory, a hard disk, a magnetic/optical medium, or a slid-state drive (SSD)). Further, the computer-readable media may be predetermined available media accessible by the computer and for example, includes all of volatile and non-volatile media and removable and irremovable media.

Further, the device and the method for reconstructing the 3D oral scan data according to the embodiments of the present invention may include instructions executable in whole or in part by the computer, and the computer program may include programmable machine instructions processed by a processor, and may be implemented by a high-level programming language, an object-oriented programming language, an assembly language, or a machine language.

As above, preferred embodiments of the invention have been described and illustrated using specific terminology, but such terminology is only intended to clearly describe the present invention. In addition, it is apparent that various modifications and changes can be made to the embodiments and described terms of the present invention without departing from the technical spirit and scope of the following claims. These modified embodiments should not be understood individually from the spirit and scope of the present invention, but should be regarded as falling within the scope of the claims of the present invention.

[Explanation of Reference Numerals and Symbols]

1: Oral scanner
2: CT apparatus
3a, 3b: 2D point
4a, 4b: 3D point
4b': Two adjacent 3D points
5a, 5b: 3D feature point
6: Points on 3D coordinate information of CT image
CP: Center point
10: 3D oral scan data reconstruction device
11: Scan data acquisition unit
12: CT image acquisition unit
13: 3D coordinate information generation unit
14: 3D feature point detection unit
15: Coordinate matching unit
16: Rematching and rigid transform calculation unit
17: Oral scan model reconstruction unit

**Claims**

1. A method for reconstructing 3D oral scan data by using a computed tomography (CT) image and scan data of which coordinates are matched based on 3D feature points of the detected CT image and the scan data, the method comprising:

   (a) a process of matching scan key frames of the scan data initially positioned by the 3D feature points, and obtaining a rigid transform of the scan key frames to minimize a 3D distance between the scan key frames and the CT image; and
   (b) a process of reconstructing a 3D oral scan model by correcting and rematching 3D coordinate information of an original scan key frame by using the obtained rigid transform of the scan key frames.

2. The method of claim 1, wherein in the process (a), if the number of scan frames for a scan frame set $S = \{s_1, s_2, \cdots\}$ is $|S|$, a rigid transform $\{T_i\}_{i=1}^{|S|}$ of each scan frame that minimizes a loss L is obtained as in [Equation] below:

EP 4 481 671 A1

[Equation]

$$L\left(\{T_i\}_{i=1}^{iS}\right) = \sum_{i=1}^{iS} w_{1,i} \sum_{j=1}^{iS} \sum_{(k_1,l)\in C(i,j)} \|Tp_{i,k_1} - Tp_{i,l}\|^2 + \sum_{i=1}^{iS} w_{2,i} \sum_{k_2=0}^{N_i} \|Tp_{i,k_2} - c_{i,k_2}\|^2$$

wherein $p_{i,k}$, represents a point corresponding an $i$-th scan key frame, $C(i,j)$ represents a set of all points that are shared between an $i$-th key frame and a $j$-th key frame, $c_{i,k}$ represents a point of 3D coordinate information of the CT image corresponding to $p_{i,k}$, $N_i$ represents the number of points where $p_{i,k}$, and $c_{i,k}$ correspond to each other, and $w_{1,i}$ and $w_{2,i}$ represent weights corresponding to respective losses.

3. The method of claim 2, wherein in the process (a), when $w_{1,i}$ becomes larger upon matching the scan key frames of the scan data, matching the scan key frames is emphasized, and when $w_{2,i}$ becomes larger, matching the scan key frame and the CT image is emphasized.

4. The method of claim 2, wherein in the process (a), by comparison with a rigid transform $T_i$ in which $L(T_i)$ is minimal with respect to fixed $w_{1,i}$ and $w_{2,i}$, a rigid transform obtained by increasing only $w_{1,i}$ decreases a matching error between scan key frames instead of increasing the matching error between the scan key frame and the CT image, and in contrast, a rigid transform obtained by increasing only $w_{2,i}$ decreases the matching error between the scan key frame and the CT image instead of increasing the matching error between the scan key frames.

5. The method of claim 2, wherein in the process (a), when artifact is severe in the CT image, a size of $w_{2,i}$ is partially decreased or is set to 0, and a size of $w_{1,i}$ is increased to reduce an influence of the CT image when matching the scan key frames, and in contrast, when the matching error between the scan key frames is large, the size of $w_{1,i}$ is decreased or is 0, and the size of $w_{2,i}$ is increased to increase the influence of the CT image, thereby reducing the matching error between the scan key frames.

6. A method for reconstructing 3D oral scan data by using a computed tomography image, the method comprising:

(a) a process of generating 3D coordinate information from a computed tomography (CT) image;
(b) a process of detecting the 3D coordinate information of the CT image and a 3D feature point of scan data;
(c) a process of matching the 3D coordinate information of the CT image and coordinates of the scan data by using the 3D feature points;
(d) a process of matching scan key frames of the scan data initially positioned by the 3D feature points, and obtaining a rigid transform of the scan key frames to minimize a 3D distance between the scan key frames and the CT image; and
(e) a process of reconstructing a 3D oral scan model by correcting and rematching 3D coordinate information of an original scan key frame by using the obtained rigid transform of the scan key frames.

7. The method of claim 6, wherein the step (b) above includes

(b-1) a process of generating a 2D rendering image for the 3D coordinate information of the scan data and the CT image;
(b-2) a process of detecting adjacent 2D points between teeth in the 2D rendering image;
(b-3) a process of detecting adjacent 3D points between teeth based on the detected 2D points; and
(b-4) a process of obtaining a direction perpendicular to a virtual straight line passing through the two adjacent detected 3D points, and obtaining a center point which becomes a center between the two adjacent 3D points in the obtained direction perpendicular to the virtual straight line, and then sampling points on the 3D coordinate information in the perpendicular direction obtained from the two adjacent 3D points and the center point to detect the 3D coordinate information of the CT image and the 3D feature point of the scan data.

8. The method of claim 6, wherein in the process (c), a rotation matrix R and a translation vector t are obtained by using [Equation 1] below by using the detected 3D feature points as an initial value, and coordinates of the scan data and the CT image are approximately matched, and the coordinates of the scan data and the CT image are matched by using [Equation] below with respect to all 3D coordinate information generated by the CT image and all points of the scan data again to minimize errors:

[Equation]

$$E(R,t) = \|C - (RD + t)\|$$

wherein 'C' represents the 3D coordinate information generated from the CT image, and 'D' represents the scan data.

**9.** The method of claim 6, wherein in the process (c), if the numbers of the 3D feature points of the scan data and the CT image are n with respect to all 3D coordinate information C and scan data D generated by the CT image, the scan data is transformed into $R_1 D + t_1$ by obtaining an initial rigid transform $R_1$, $t_1$ of minimizing the error $E(R_1, t_1)$ by using [Equation 1] below with respect to the 3D feature points $\{c_i\}_{i=0}^{n} \subseteq C$ of the CT image and the 3D feature points $\{d_i\}_{i=0}^{n} \subseteq D$ of the scan data to approximately match the coordinates of the scan data and the CT image, and coordinate information of the scan data D is transformed into $R(R_1 D + t_1) + t$ by obtaining the rigid transform R,t of minimizing the error $E(R,t)$ by using [Equation 2] below with respect to 3D coordinate information C generated by the CT image and all points $R_1 D + t_1$ of coordinate-shifted scan data to match the coordinates of the scan data and the CT image:

[Equation 1]

$$E(R_1, t_1) = \sum_{i=0}^{n} \|c_i - (R_1 d_i + t_1)\|$$

[Equation 2]

$$E(R,t) = \|C - (R(R_1 D + t_1) + t)\|$$

**10.** The method of claim 6, wherein in the process (d), if the number of scan frames for a scan frame set $S = \{s_1, s_2, \cdots\}$ is $|S|$, a rigid transform $\{T_i\}_{i=1}^{|S|}$ of each scan frame that minimizes a loss L is obtained as in [Equation] below:

[Equation]

$$L(\{T_i\}_{i=1}^{|S|}) = \sum_{i=1}^{|S|} w_{1,i} \sum_{j=1}^{|S|} \sum_{(k_i, l) \in C(i,j)} \|T_i p_{i,k_i} - T_j p_{j,l}\|^2 + \sum_{i=1}^{|S|} w_{2,i} \sum_{k_i=0}^{N_i} \|T_i p_{i,k_i} - c_{i,k_i}\|^2$$

wherein $p_{i,k}$ represents a point corresponding an $i$-th scan key frame, $C(i,j)$ represents a set of all points that are shared between an $i$-th key frame and a $j$-th key frame, $C_{i,k}$ represents a point of 3D coordinate information of the CT image corresponding to $p_{i,k}$, $N_i$ represents the number of points where $p_{i,k}$ and $c_{i,k}$ correspond to each other, and $w_{1,i}$ and $w_{2,i}$ represent weights corresponding to respective losses.

**11.** The method of claim 10, wherein in the process (d), when $w_{1,i}$ becomes larger upon matching the scan key frames of the scan data, matching the scan key frames is emphasized, and when $w_{2,i}$ becomes larger, matching the scan key frame and the CT image is emphasized.

**12.** The method of claim 10, wherein in the process (d), by comparison with a rigid transform $T_i$ in which $L(T_i)$ is minimal with respect to fixed $w_{1,i}$ and $w_{2,i}$, in the same condition, a rigid transform obtained by increasing only $w_{1,i}$ decreases a matching error between the scan key frames instead of increasing the matching error between the scan key frame and the CT image, and in contrast, a rigid transform obtained by increasing only $w_{2,i}$ decreases the matching error between the scan key frame and the CT image instead of increasing the matching error between the scan key frames.

13. The method of claim 10, wherein in the process (d), when artifact is severe in the CT image, a size of $w_{2,i}$ is partially decreased or is set to 0, and a size of $w_{1,i}$ is increased to reduce an influence of the CT image when matching the scan key frames, and in contrast, when the matching error between the scan key frames is large, the size of $w_{1,i}$ is decreased or is 0, and the size of $w_{2,i}$ is increased to increase the influence of the CT image, thereby reducing the matching error between the scan key frames.

14. A device for reconstructing 3D oral scan data by using a computed tomography image, the device comprising:

a 3D coordinate information generation unit generating 3D coordinate information from a computed tomography (CT) image;
a 3D feature point detection unit detecting the 3D coordinate information of the CT image and a 3D feature point of scan data;
a coordinate matching unit matching the 3D coordinate information of the CT image and coordinates of the scan data by using the 3D feature points;
a rematching and rigid transform calculation unit matching scan key frames of the scan data initially positioned by the 3D feature points, and obtaining a rigid transform of the scan key frames to minimize a 3D distance between the scan key frames and the CT image; and
an oral scan model reconstruction unit reconstructing a 3D oral scan model by correcting and rematching 3D coordinate information of an original scan key frame by using the obtained rigid transform of the scan key frames.

15. The device of claim 14, wherein the 3D feature point detection unit generates a 2D rendering image for the 3D coordinate information of the scan data and the CT image, detects adjacent 2D points between teeth in the generated 2D rendering image, detects adjacent 3D points between teeth based on the detected 2D points, and obtains a direction perpendicular to a virtual straight line passing through the two adjacent detected 3D points, and obtains a center point which becomes a center between the two adjacent 3D points in the obtained direction perpendicular to the virtual straight line, and then samples points on the 3D coordinate information in the perpendicular direction obtained from the two adjacent 3D points and the center point to detect the 3D coordinate information of the CT image and the 3D feature point of the scan data.

16. The device of claim 14, wherein the coordinate matching unit obtains a rotation matrix R and a translation vector t by using [Equation] below by using the detected 3D feature points as an initial value, and approximately matches coordinates of the scan data and the CT image, and matches the coordinates of the scan data and the CT image by using [Equation] below with respect to all 3D coordinate information generated by the CT image and all points of the scan data again to minimize errors:

[Equation]

$$E(R,t) = \|C - (RD + t)\|$$

wherein 'C' represents the 3D coordinate information generated from the CT image, and 'D' represents the scan data.

17. The device of claim 14, wherein if the numbers of the 3D feature points of the scan data and the CT image are n with respect to all 3D coordinate information C and scan data D generated by the CT image, the coordinate matching unit transforms the scan data into $R_1D+t_1$ by obtaining an initial rigid transform $R_1$, $t_1$ of minimizing the error $E(R_1,t_1)$ by using [Equation 1] below with respect to the 3D feature points $\{c_i\}_{i=0}^{n} \subseteq C$ of the CT image and the 3D feature points $\{d_i\}_{i=0}^{n} \subseteq D$ of the scan data to approximately match the coordinates of the scan data and the CT image, and transforms the coordinate information of the scan data D into $R(R_1D+t_1) + t$ by obtaining the rigid transform R,t of minimizing the error $E(R,t)$ by using [Equation 2] below with respect to 3D coordinate information C generated by the CT image and all points $R_1D+t_1$ of coordinate-shifted scan data to match the coordinates of the scan data and the CT image:

[Equation 1]

$$E(R_1, t_1) = \sum_{i=0}^{n} \| c_i - (R_1 d_i + t_1) \|$$

[Equation 2]

$$E(R, t) = \| C - (R(R_1 D + t_1) + t) \|$$

18. The device of claim 14, wherein if the number of the scan frames for a scan frame set $S = \{s_1, s_2, \cdots \}$ is $|S|$, the rematching and rigid transform calculation unit obtains a rigid transform $\{T_i\}_{i=1}^{|S|}$ of each scan frame that minimizes a loss L as in [Equation] below:

[Equation]

$$L(\{T_i\}_{i=1}^{|S|}) = \sum_{i=1}^{|S|} w_{1,i} \sum_{j=1}^{|S|} \sum_{(k_1, l) \in C(i,j)} \| T_i p_{i,k_1} - T_j p_{j,l} \|^2 + \sum_{i=1}^{|S|} w_{2,i} \sum_{k_2=0}^{N_i} \| T_i p_{i,k_2} - c_{i,k_2} \|^2$$

wherein $p_{i,k}$ represents a point corresponding an $i$-th scan key frame, $C(i,j)$ represents a set of all points that are shared between an $i$-th key frame and a $j$-th key frame, $C_{i,k}$ represents a point of 3D coordinate information of the CT image corresponding to $p_{i,k}$, $N_i$ represents the number of points where $p_{i,k}$ and $c_{i,k}$ correspond to each other, and $w_{1,i}$ and $w_{2,i}$ represent weights corresponding to respective losses.

19. The device of claim 18, wherein the rematching and rigid transform calculation unit emphasizes matching the scan key frames when $w_{1,i}$ becomes larger upon matching the scan key frames of the scan data, and emphasizes matching the scan key frame and the CT image when $w_{2,i}$ becomes larger.

20. The device of claim 18, wherein by comparison with a rigid transform $T_i$ in which $L(T_i)$ is minimal with respect to fixed $w_{1,i}$ and $w_{2,i}$, in the same condition, the rematching and rigid transform calculation unit decreases a matching error between the scan key frames instead of increasing the matching error between the scan key frame and the CT image in a rigid transform obtained by increasing only $w_{1,i}$, and in contrast, decreases the matching error between the scan key frame and the CT image instead of increasing the matching error between the scan key frames in a rigid transform obtained by increasing only $w_{2,i}$.

21. The device of claim 18, wherein the rematching and rigid transform calculation unit partially decreases a size of $w_{2,i}$ or sets the size of $w_{2,i}$ to 0, and increases a size of $w_{1,i}$ to reduce an influence of the CT image when matching the scan key frames when artifact is severe in the CT image, and in contrast, decreases the size of $w_{1,i}$ or set the size of $w_{1,i}$ to 0, and increases the size of $w_{2,i}$ to increase the influence of the CT image when the matching error between the scan key frames is large, thereby reducing the matching error between the scan key frames.

22. An oral scanner including the device for reconstructing 3D oral scan data by using a computed tomography image of any one of claims 14 to 21.

23. A computed tomography (CT) apparatus including the device for reconstructing 3D oral scan data by using a computed tomography image of any one of claims 14 to 21.

24. A computer-readable recording medium storing a program for implementing the method for reconstructing 3D oral scan data by using a computed tomography image of any one of claims 1 to 13.

25. A computer program stored in a computer-readable recording medium for implementing the method for reconstructing 3D oral scan data by using a computed tomography image of any one of claims 1 to 13.

FIG. 1

10

| 11 | | 14 | 15 |
|---|---|---|---|

SCAN DATA
ACQUISITION UNIT

3D FEATURE POINT
DETECTION UNIT

COORDINATE
MATCHING UNIT

CT IMAGE
ACQUISITION UNIT

3D COORDINATE INFORMATION
GENERATION UNIT

12

13

REMATCHING AND
RIGID TRANSFORM CALCULATION UNIT

16

ORAL SCAN MODEL
RECONSTRUCTION UNIT

17

FIG. 2

```
                    ╭─────────╮
                    │  START  │
                    ╰─────────╯
                         │
                         ▼
    ┌─────────────────────────────────────────────────┐
    │  ACQUIRE SCAN DATA AND CT IMAGE (DATA)           │───── S1
    └─────────────────────────────────────────────────┘
                         │
                         ▼
    ┌─────────────────────────────────────────────────┐
    │ GENERATE 3D COORDINATE INFORMATION OF CT IMAGE   │
    │   (DEEP LEARNING UPPER/LOWER JAW DIVISION)       │───── S2
    └─────────────────────────────────────────────────┘
                         │
                         ▼
    ┌─────────────────────────────────────────────────┐
    │          DETECT 3D FEATURE POINT                 │
    │ FOR 3D COORDINATE INFORMATION OF CT IMAGE AND    │───── S3
    │                SCAN DATA                         │
    └─────────────────────────────────────────────────┘
                         │
                         ▼
    ┌─────────────────────────────────────────────────┐
    │  MATCH COORDINATES OF SCAN DATA AND CT IMAGE     │───── S4
    └─────────────────────────────────────────────────┘
                         │
                         ▼
    ┌─────────────────────────────────────────────────┐
    │          REMATCH SCAN KEY FRAMES                 │
    │        AND OBTAIN RIGID TRANSFORM                │───── S5
    └─────────────────────────────────────────────────┘
                         │
                         ▼
    ┌─────────────────────────────────────────────────┐
    │       RECONSTRUCT 3D ORAL SCAN MODEL             │───── S6
    └─────────────────────────────────────────────────┘
                         │
                         ▼
                    ╭─────────╮
                    │   END   │
                    ╰─────────╯
```

FIG. 3

ORAL SCANNER(1)

SCAN DATA

3D FEATURE POINT

CT APPARATUS(CBCT)(2)

CT IMAGE

UPPER/LOWER JAW DIVISION AND
3D COORDINATE INFORMATION GENERATION

FIG. 4

S3

'S2'

| GENERATE 2D RENDERING IMAGE BY RENDERING 3D COORDINATE INFORMATION OF SCAN DATA AND CT IMAGE IN Z-AXIS DIRECTION |
| --- |

S31

| DETECT ADJACENT POINTS (2D POINTS) BETWEEN TEETH IN 2D RENDERING IMAGE (DEEP LEARNING NETWORK LEARNING) |
| --- |

S32

| DETECT ADJACENT POINTS (3D POINTS) BETWEEN TEETH BY USING 2D POINTS (PROJECTION) |
| --- |

S33

| DETECT 3D FEATURE POINTS BY USING 3D POINTS |
| --- |

S34

'S4'

FIG. 5

2D RENDERING IMAGE    2D ADJACENT POINTS BETWEEN TEETH    3D ADJACENT POINTS BETWEEN TEETH    3D FEATURE POINT

SCAN DATA    Deep learning

CT IMAGE (DATA)

FIG. 6

FIG. 7

FIG. 8

FIG. 9

FIG. 10

SCAN CAMERA DIRECTION

SCAN DATA          CT IMAGE

<div align="center">

### INTERNATIONAL SEARCH REPORT

</div>

| | International application No. |
|---|---|
| | **PCT/KR2022/019837** |

**A. CLASSIFICATION OF SUBJECT MATTER**

**G06T 5/00**(2006.01)i; **G06T 7/33**(2017.01)i; **G06T 7/73**(2017.01)i; **G06T 11/00**(2006.01)i; **A61B 6/00**(2006.01)i; **A61B 6/14**(2006.01)i; **A61B 6/03**(2006.01)i; **A61B 5/00**(2006.01)i; **A61C 7/00**(2006.01)i

According to International Patent Classification (IPC) or to both national classification and IPC

**B. FIELDS SEARCHED**

Minimum documentation searched (classification system followed by classification symbols)

G06T 5/00(2006.01); A61B 17/56(2006.01); A61B 5/00(2006.01); A61B 6/03(2006.01); A61C 9/00(2006.01); G06T 7/30(2017.01); G06T 7/593(2017.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 컴퓨터 단층촬영(computed tomography, CT), 영상(image), 3차원(3-dimensional), 구강(oral), 스캔(scan), 복원(reconstruction), 특징점(feature point), 좌표(coordinate), 매칭(matching), 키 프레임(key frame), 거리(distance), 강체 변환(rigid transform), 보정(correction)

**C. DOCUMENTS CONSIDERED TO BE RELEVANT**

| Category* | Citation of document, with indication, where appropriate, of the relevant passages | Relevant to claim No. |
|---|---|---|
| DY | KR 10-2273437 B1 (HDX WILL CORP.) 07 July 2021 (2021-07-07)<br>See paragraph [0034]; and claim 1. | 1,6,14,22-25 |
| DA | | 2-5,7-13,15-21 |
| Y | KR 10-2273438 B1 (HDX WILL CORP.) 07 July 2021 (2021-07-07)<br>See claim 1. | 1,6,14,22-25 |
| Y | KR 10-1954487 B1 (ALIGN TECHNOLOGY, INC.) 05 March 2019 (2019-03-05)<br>See claim 8. | 1,6,14,22-25 |
| A | ZHOU, Xinwen et al. A Method for Tooth Model Reconstruction Based on Integration of Multimodal Images. Journal of Healthcare Engineering. Volume 2018, Article ID 4950131, pp. 1-8, 20 June 2018.<br>See pages 1-7. | 1-25 |

☑ Further documents are listed in the continuation of Box C.　　☑ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
|---|---|
| **15 March 2023** | **16 March 2023** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2022)

| | International application No. |
|---|---|
| | **PCT/KR2022/019837** |

**C. DOCUMENTS CONSIDERED TO BE RELEVANT**

| Category* | Citation of document, with indication, where appropriate, of the relevant passages | Relevant to claim No. |
|---|---|---|
| A | WO 2015-142291 A1 (NATIONAL UNIVERSITY OF SINGAPORE) 24 September 2015 (2015-09-24) See claims 10, 12 and 14. | 1-25 |
| PX | KR 10-2453897 B1 (HDX WILL CORP.) 12 October 2022 (2022-10-12) See paragraphs [0010]-[0057]; and claims 1, 3-6, 8-14 and 16-25. * This document is a published earlier application that serves as a basis for claiming priority of the present international application. | 1-25 |

INTERNATIONAL SEARCH REPORT
Information on patent family members

International application No.

**PCT/KR2022/019837**

| Patent document cited in search report | | | Publication date (day/month/year) | Patent family member(s) | | | Publication date (day/month/year) |
|---|---|---|---|---|---|---|---|
| KR | 10-2273437 | B1 | 07 July 2021 | WO | 2022-158804 | A1 | 28 July 2022 |
| KR | 10-2273438 | B1 | 07 July 2021 | WO | 2022-164126 | A1 | 04 August 2022 |
| KR | 10-1954487 | B1 | 05 March 2019 | AU | 2013-340464 | A1 | 07 May 2015 |
| | | | | AU | 2013-340464 | B2 | 08 June 2017 |
| | | | | CN | 104837436 | A | 12 August 2015 |
| | | | | CN | 104837436 | B | 08 March 2017 |
| | | | | EP | 2914202 | A2 | 09 September 2015 |
| | | | | EP | 2914202 | B1 | 07 December 2016 |
| | | | | ES | 2615740 | T3 | 08 June 2017 |
| | | | | IL | 238304 | A | 30 June 2015 |
| | | | | IL | 238304 | B | 31 December 2018 |
| | | | | JP | 2016-500547 | A | 14 January 2016 |
| | | | | JP | 6344824 | B2 | 20 June 2018 |
| | | | | KR | 10-2015-0082428 | A | 15 July 2015 |
| | | | | US | 10275862 | B2 | 30 April 2019 |
| | | | | US | 10600160 | B2 | 24 March 2020 |
| | | | | US | 11321817 | B2 | 03 May 2022 |
| | | | | US | 2014-0120493 | A1 | 01 May 2014 |
| | | | | US | 2015-0178901 | A1 | 25 June 2015 |
| | | | | US | 2016-0104268 | A1 | 14 April 2016 |
| | | | | US | 2017-0140511 | A1 | 18 May 2017 |
| | | | | US | 2018-0096465 | A1 | 05 April 2018 |
| | | | | US | 2019-0244332 | A1 | 08 August 2019 |
| | | | | US | 2020-0202497 | A1 | 25 June 2020 |
| | | | | US | 2022-0207660 | A1 | 30 June 2022 |
| | | | | US | 8948482 | B2 | 03 February 2015 |
| | | | | US | 9214014 | B2 | 15 December 2015 |
| | | | | US | 9589329 | B2 | 07 March 2017 |
| | | | | US | 9830688 | B2 | 28 November 2017 |
| | | | | WO | 2014-068383 | A2 | 08 May 2014 |
| | | | | WO | 2014-068383 | A3 | 07 August 2014 |
| | | | | WO | 2014-068383 | A8 | 08 May 2014 |
| WO | 2015-142291 | A1 | 24 September 2015 | None | | | |
| KR | 10-2453897 | B1 | 12 October 2022 | None | | | |

Form PCT/ISA/210 (patent family annex) (July 2022)

**EP 4 481 671 A1**

**Patent documents cited in the description**

- KR 102273437 B1